# EUROPEAN PATENT SPECIFICATION

(11) **EP 3 146 930 B1**
(45) Date of publication and mention of the grant of the patent: **06.06.2018**
(21) Application number: 15186289.3
(22) Date of filing: 22.09.2015
(51) Int. Cl.: A61B 34/30

(54) **A SURGICAL ROBOT'S TOOL ARMS ASSEMBLY**
WERKZEUGARMANORDNUNGEN EINES CHIRURGISCHEN ROBOTERS
ENSEMBLE DE BRAS D'OUTIL DE ROBOT CHIRURGICAL

(43) Date of publication of application: 29.03.2017
(73) Proprietor: Fundacja Rozwoju Kardiochirurgii Im. Prof. Zbigniewa Religi, 41-800 Zabrze (PL)
(72) Inventor: Nawrat, Zbigniew, 41-800 Zabrze (PL); Mucha, Lukasz, 37-700 Przemysl (PL); Lehrich, Krzysztof, 43-450 Ustron (PL); Lis, Krzysztof, 42-674 Wilkowice (PL); Rohr, Kamil, 41-933 Bytom (PL)
(74) Representative: Malcherek, Piotr

(56) References cited:
- EP-A1- 1 681 029
- WO-A1-2014/151621
- WO-A2-01/97680
- US-A- 5 749 362
- US-A1- 2014 180 309

## Description

This invention relates to a surgical robot's tool arms assembly provided as an element of a single support arm of the robot equipping said robot with surgical instruments necessary to carry out a specific medical procedure. The tool arms' assembly connected with the surgical robot's support arm enables the execution of minimally invasive surgical procedures and medical procedures which require high precision, also those remotely controlled, carried out with the use of telemanipulation.

### STATE OF THE ART

In the prior are there are known devices which enable mounting surgical tools and endoscopic cameras on a single support arm of a surgical robot. For example, WO2010/068005 discloses a solution relating to a surgical robot adapted to be remotely controlled, wherein said robot has a base on which the support arm is mounted. There are a number of tool arms connected to the support arm, wherein each tool arm has a surgical instrument at its end. It is possible to connect rotatably a second additional support arm to the first support arm. Moreover, it is possible to provide a surgical robot, wherein subsequent intermediate tool arms are connected to the support arm of the robot and the last of said tool arms is equipped with a surgical instrument at its end.

Moreover, KR20120042344 discloses a surgical robot's support arm comprising a first arm mounted on a base on one side, and on the other side movably connected to a second arm, in which a first movable bracket is mounted. On said first movable bracket a number of other movable brackets may be slidably mounted and surgical instruments can be connected thereto.

### AIM OF THE INVENTION

The aim of the invention is to provide a universal tool arms assembly of a surgical robot equipped with surgical instruments and fixing elements for a single support arm of any surgical robot, which should first of all eliminate low functionality of a surgical robot's single support arm usually equipped with only one surgical instrument, and furthermore eliminate problems resulting from limited work area and the possibility of collision occurring when surgical robots with multi-instrument arm assemblies are used. Another aim of the invention is to provide an extended range of work of each of the tool arms with surgical tools mounted thereon and to enable distribution of said instruments in remote points within the operating field. Still another aim of the invention is to extend the space around a patient accessible both for a surgeon and for other medical devices by means of reducing the space and weight of the surgical robot's tool arms assembly. Moreover, the aim of the invention is also to eliminate problems resulting from accumulating instruments in a narrow work area by eliminating the need to use one common trocar for all surgical instruments.

### THE ESSENCE OF THE INVENTION

The invention relates to a surgical robot's tool arms assembly comprising three movable tool arms mounted on a base provided for detachable fixing thereof on the surgical robot's support structure. The essence of the invention consists in that the first of the tool arms is mounted centrally on the base by means of a rotatable joint and is equipped with a first guide rail with at least one arch-shaped guide groove and a second guide rail slides in said guide groove, and in the second guide rail there is also at least one arch-shaped guide groove in which slides a third guide rail with an arch-like profile in a side view and said third guide rail is connected to a base for fixing a surgical instrument. At the same time the mutual position of all the three guide rails is determined with a guiding and locking element, preferably a screw mounted in the third guide rail and going through the guide grooves and equipped with a clamp. Each of the remaining two tool arms is located at opposite ends of the first tool arm and connected to the base by means of a first platform rotatably mounted on the base. Each of said two tool arms comprises a slidably and rotatably connected to the first platform guide rail with a second platform slidably and rotatably mounted thereon, wherein said second platform is locked on the guide rail with a locking element, preferably a screw. On the second platform there is an immovably mounted first motor and to the shaft thereof there is mounted a first pivot bracket at the second end of which there is an immovably mounted second motor and to the shaft thereof is mounted a second pivot bracket provided for mounting a surgical instrument thereon.

It is preferable that the clamp of the screw locking the guide rails be equipped with eccentrically mounted therein roller elements.

It is advisable that the second pivot bracket be connected with a linear slide mechanism on which a surgical instrument is mounted, wherein both axes of rotation of the first and of the second motor intersect at one point with an axis of rotation of the surgical instrument.

It is recommendable that a fixing base have a form of a semi-open profile wherein the surgical instrument is slidably mounted.

In a preferred embodiment the surgical instrument fixed to the fixing base of the first tool arm has a form of a video system body along which slides a tool slide with a camera.

It is particularly advisable that the first platform be equipped with a third motor provided for a rotatable movement of the platform or the guide rail mounted thereon.

In a preferred embodiment the second platform is equipped with a fourth motor provided for rotatable movement and moving the platform along the guide rail.

It is also recommendable that an intermediate platform be mounted on the second platform and a slidable platform with the immovably mounted thereon first motor be connected to said intermediate platform.

It is particularly recommendable that the intermediate platform be equipped with a fifth motor provided for reciprocating movement of the slidable platform.

### ADVANTAGES OF THE INVENTION

The main advantage of the invention is a substantial enhancement in the functionality of a surgical robot's single support arm, and at the same time eliminating the possibility of collision of the tool arms assembly as well as the possibility of collision of the tool arms assembly with the surgical robot's support arm and also the possibility of collision between the tool arms assembly and the movable elements of the surgical instruments mounted thereon. While enhancing the functionality of the surgical robot's single arm and eliminating the possibility of collision, a low weight of the tool arms assembly was maintained and a wide scope of movement of each tool arm and surgical instruments mounted thereon was ensured, which is another advantage of the invention. Yet another advantage of the invention is the possibility to preset each tool arm with the surgical instrument mounted thereon, while maintaining the stable position of the surgical robot's support arm. Moreover, it is also possible to compensate the movements of the support arm with the tool arms of the assembly, which enables to maintain constant-pointedness of the selected tool arms while moving the support arm at the same time. Another advantage of the invention is that the tool arms assembly leaves substantial space for accessing the patient by both a surgeon and other medical devices.

### DESCRIPTION OF THE DRAWINGS

The invention is presented in more detail in embodiments and in the drawings, where:

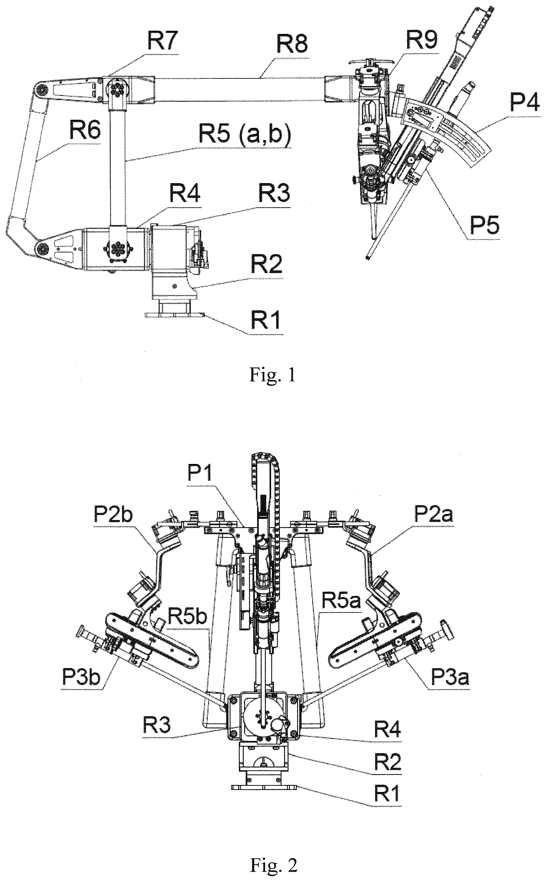

- Fig. 1: is a side view of the tool arms assembly mounted on the surgical robot's support arm,
- Fig. 2: is a front view of the tool arms assembly mounted on the surgical robot's support arm,

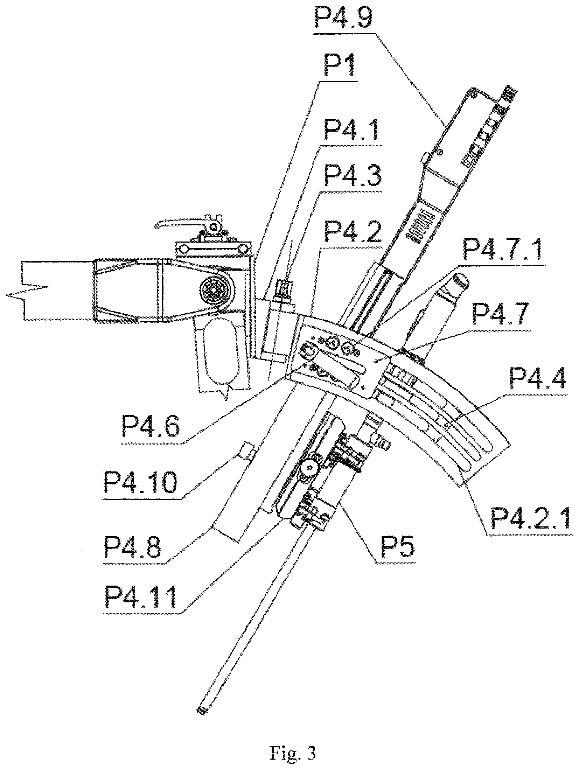

- Fig. 3: is a side view of the first tool arm,

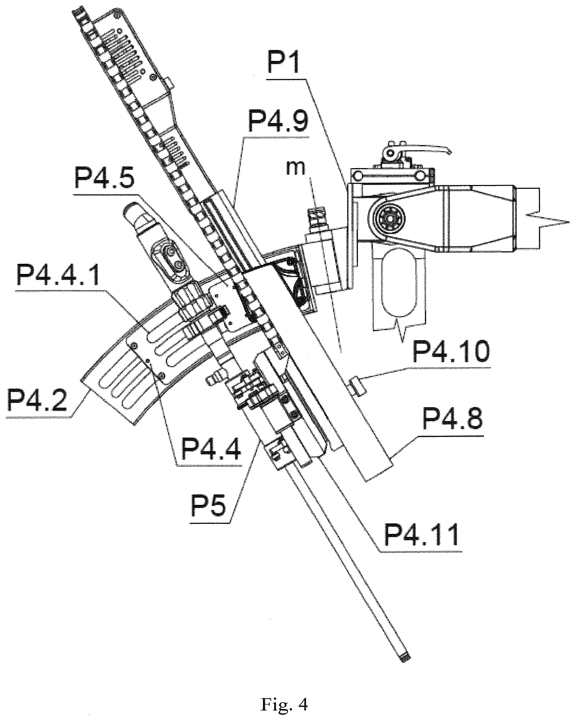

- Fig. 4: is an alternative side view of the first tool arm,

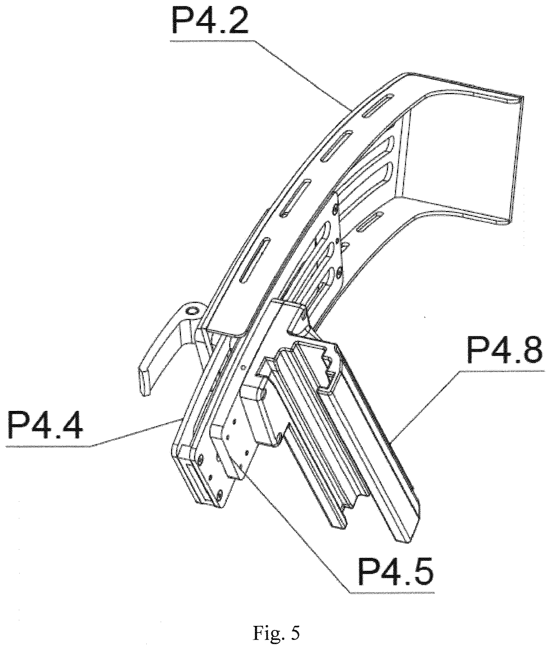

- Fig. 5: is a perspective view of a part of the first tool arm,

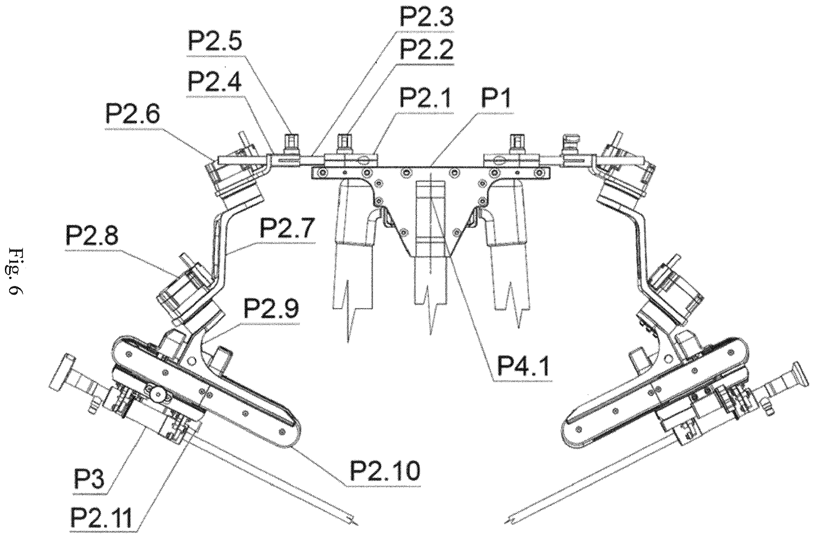

- Fig. 6: is a front view of the two lateral tool arms,

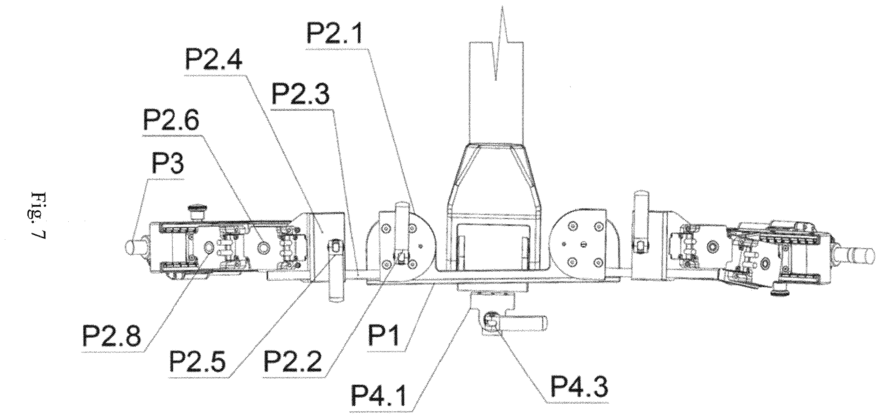

- Fig. 7: is a top view of the two lateral tool arms,

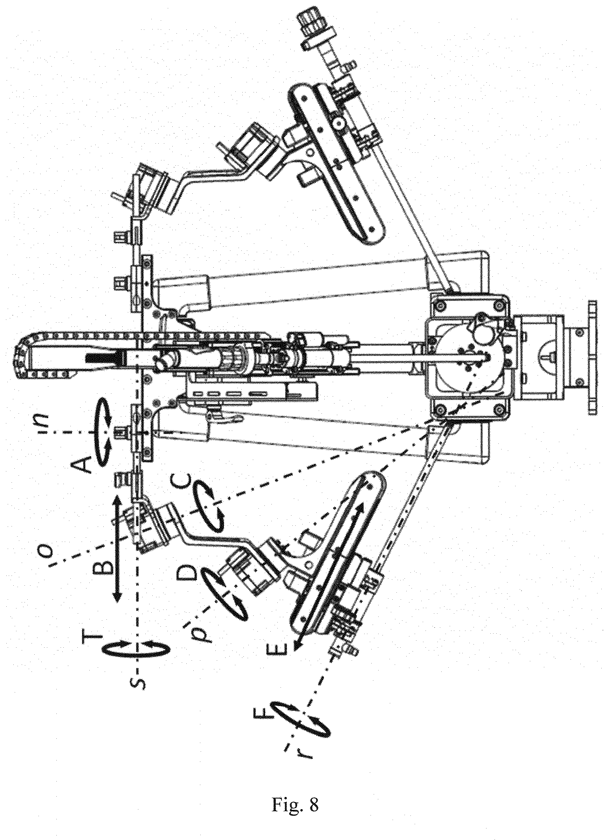

- Fig. 8: is a front view of the tool arms assembly mounted on the surgical robot's support arm with indicated directions of movement of the lateral tool arm,

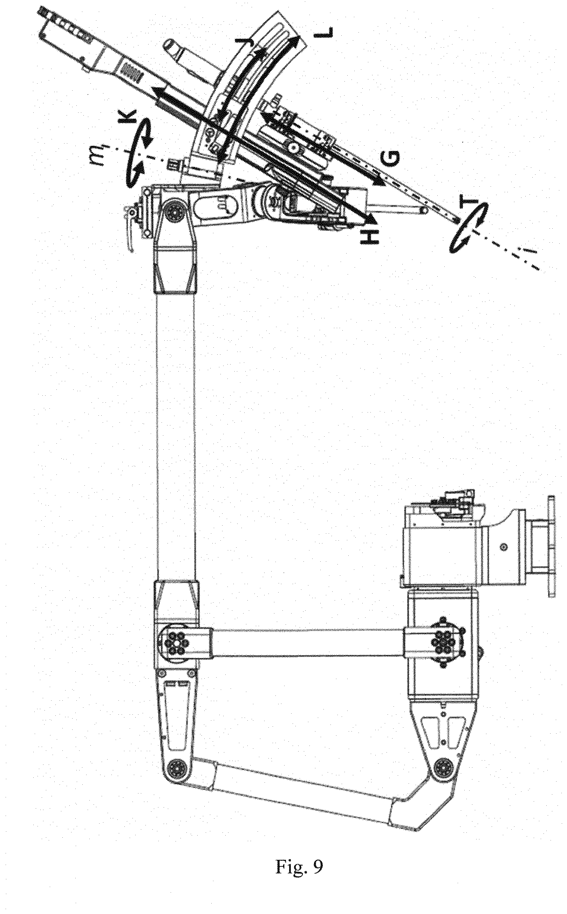

- Fig. 9: is a side view of the tool arms assembly mounted on the surgical robot's support arm with indicated directions of movement of the first tool arm,

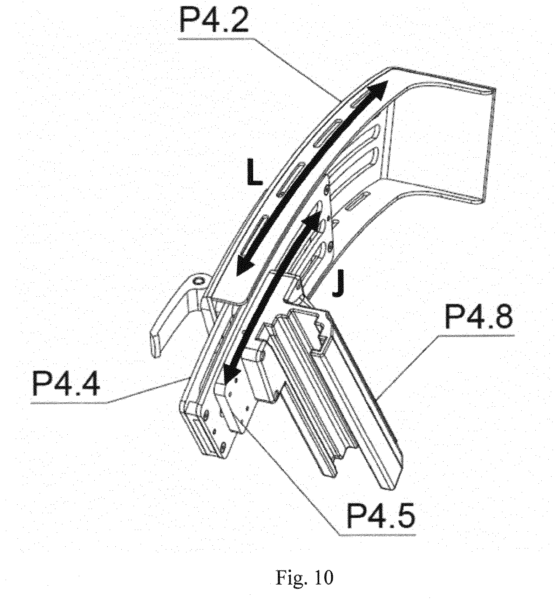

- Fig. 10: is a perspective view of a part of the first tool arm with indicated directions of movement,

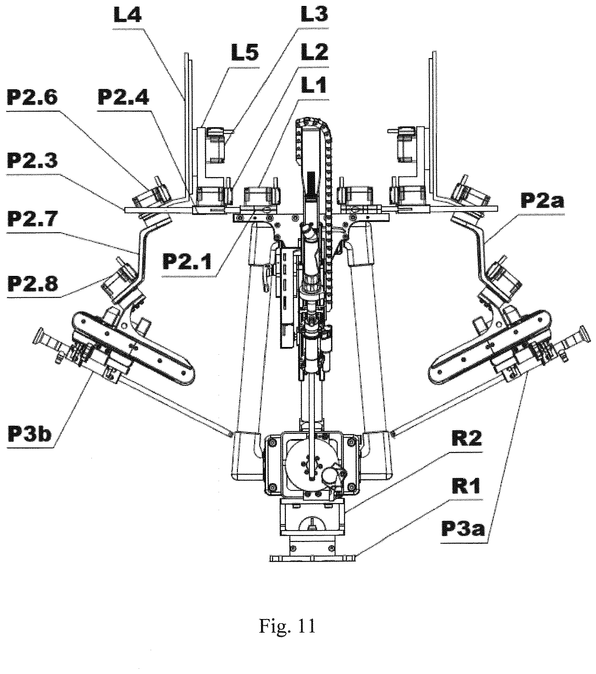

- Fig. 11: is a front view of the tool arms assembly mounted on the surgical robot's support arm in an alternative embodiment,

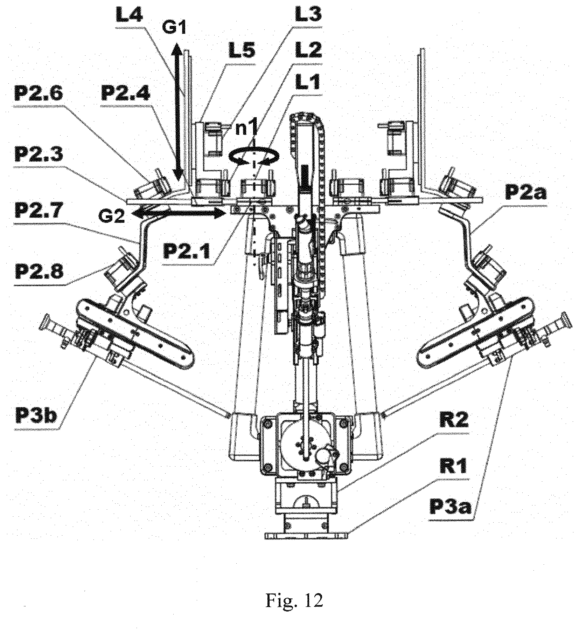

- Fig. 12: is a front view of the tool arms assembly mounted on the surgical robot's support arm with indicated directions of movement in the same embodiment as in Fig. 11

### EMBODIMENT

The surgical robot's tool arms assembly is presented in embodiments in an exemplary connection with the surgical robot's support arm which resembles a four-bar linkage. It is obvious that it is possible to mount the assembly on a support arm of a different construction.

The surgical robot's support arm is equipped with a fixing base R1 on which there is a rotatably mounted platform R2 connected with a first immovable body. Inside the immovable body there is a motor R3 and to the shaft thereof there is a rigidly mounted angular tilting body R4. Inside the angular tilting body R4 there is a motor and a shaft thereof is connected with two legs R5a, R5b located on the opposite sides of the body, wherein an axis of rotation of the shaft of the motor mounted in the angular tilting body is perpendicular to the axis of rotation of the shaft of the motor R3 located in the immovable body. Furthermore, there is a third leg R6 rotatably mounted to the angular tilting body R4. All legs R5a, R5b, R6 fixed to the angular tilting body R4 provide support for an upper body R7 within which said legs are rotatably mounted. To the upper body R7 a longitudinal guide body R8 is connected with a first end thereof, and at a second end thereof there is a pivot mechanism R9 detachably connected to a base P1 of the tool arms assembly. Inside the guide body R8 there are, not shown in detail, drive mechanisms connected with the pivot mechanism R9.

The base P1 of the tool arms assembly is equipped with three tool arms - the first tool arm P4 is centrally mounted on the base and two lateral tool arms P2a, P2b located on the opposite sides of the first tool arm P4. The first tool arm P4 is centrally mounted on the base P1 by means of a rotatable joint P4.3 and the first part P4.1 thereof is directly connected with the base P1. Moreover, on the rotatable joint P4.3 there is an element locking the position thereof. The first tool arm P4 is equipped with a first guide rail P4.2 having three arch-shaped guide grooves P4.2.1 in which slides a second guide rail P4.4 also having three arch-shaped grooves P4.4.1 in which slides a third guide rail P4.5. Said third guide rail P4.5 is arch-shaped in side view and it is connected with a base P4.8 for fixing a surgical instrument. The mutual position of all the three guide rails P4.2, P4.4, P4.5 is set by means of a guiding and locking element, in this embodiment specifically a screw P4.6 fixed in the third guide rail P4.5 and going through the guide grooves P4.2.1, P4.4.1 and equipped with a clamp P4.7. On said clamp P4.7 there are eccentrically mounted roller elements P4.7.1 which enable play compensation between the guide rails P4.2, P4.4 moving along the arch-shaped grooves P4.2.1, P4.4.1. In the embodiment there are three guide grooves, but at least one guide grooves is sufficient for operation. The construction of the first tool arm P4 comprising three guide rails significantly increases rigidity of said arm, thus making it possible to secure any surgical instruments thereon in a stable manner. For example, to the base P4.8 having a form of a semi-open profile there is a slidably mounted video system body P4.9, wherein the position of the video system body P4.9 on the base P4.8 is locked by means of a screw P.4.10. A tool slide P4.22 with a camera P5 moves along the video system body P4.9.

Each of the other two tool arms P2a, P2b is located on the opposite side of the first tool arm P4 and connected to the base P1 by means of a first platform P2.1. rotatably mounted on the base P1. The rotation and position of the first platform P2.1 is locked with a screw P2.2. Each of said tool arms P2a, P2b comprises a guide rail P2.3 slidably and rotatably connected with the first platform P2.1, said guide rail P2.3 having a second platform P2.4 slidably and rotatably mounted thereon. The guide rail P2.3 is circular in the cross section. After setting the position of the second platform P2.4 on the guide rail P2.3 said position is locked with a screw P2.5. On the second platform P2.4 there is an immovably mounted first motor P2.6 and to the shaft thereof is mounted a first pivot bracket P2.7 and on a second end of said pivot bracket P2.7 is an immovably mounted second motor P2.8. To the shaft of the second motor P2.8 is mounted a second pivot bracket P2.9 provided for mounting thereon any surgical instrument. In this embodiment the second pivot bracket P2.9 is additionally connected with a linear slide mechanism P2.10 on which a tool slide P2.12 is mounted with a not shown drive unit of a surgical tool P3 fixed to the tool slide P2.12. At the same time axes of rotation o, p of the first and the second motor P2.4, P2.8 intersect at one point with an axis of rotation r of the surgical instrument P3.

While using the tool arms assembly during medical procedures first the position of the first tool arm P4 with the camera P5 mounted thereon is set. After releasing the lock on the rotatable joint P4.3 it is possible to slide the first guide rail P4.2 around an axis of rotation m of the joint P4.3 in direction K. Releasing the guiding and locking screw P4.6 enables moving the second guide rail P4.4 along the arch-shaped grooves P4.2.1 in direction L and moving the third guide rail P4.5 with a base P4.8 for fixing thereon the surgical instrument along arch-shaped grooves P4.4.1 in direction J. The position of the surgical instrument is changed in this manner - in this embodiment a camera P5 and the angular range of work thereof. If the guide rails P4.2, P4.4, P4.5 are folded up, the operating space during a surgical procedure can be substantially extended. Moreover, after releasing the locking screw P4.10 it is possible to slide the video system body P4.9 along the base P4.8 in direction H. It is also possible to move the camera P5 with the tool slide P4.11 in direction G and rotating the instrument around its own axis and in direction T.

The position of surgical instruments mounted on each of the arms P2a, P2b is set manually, by setting the position of a constant point of the instrument in the patient's body. After releasing the locking screw P2.2 the platform P2.1 can rotate around an axis n in direction A. the rotation of the platform P2.1 results in the rotation of the guide rail P2.3 and elements connected thereto. Furthermore, it is possible to slide in or slide out the guide rail P2.3 from the first platform P2.1 in order to set the position of the robot and ensure sufficient operating space during the procedure. In order to set the position of the second platform P2.4 with subassemblies mounted thereon the locking screw P2.5 is released, which enables moving the platform P2.4 along the guide rail P2.3 in direction B and rotation of said platform P2.4 on the guide rail P2.3 around an axis s in direction T. The motor P2.6 mounted on the second platform P2.4 rotates the first pivot bracket P2.7 and elements mounted thereon around the axis o going through the axis of rotation of the shaft of the motor P2.6 in direction C. The rotation of the shaft of the motor P2.8 mounted on the first pivot bracket P2.7 around the axis of rotation p in direction D results in rotation of the second pivot bracket P2.9 with surgical instruments mounted thereon. Furthermore, the movement of the linear slide mechanism P10 connected with the bracket P2.9 slides out the surgical instrument P3 in direction E. At the same time rotation of the surgical instrument P3 around an axis of rotation r in direction F can take place with the use of a drive on the tool slide P2.12. The movement of the elements of the tool arms P2a, P2b in directions C, D, E, F during a medical procedure enables movement of the surgical instrument P3 inside a patient's body.

In another embodiment the first platform P2.1 is equipped with a third motor L1 rotating the first platform P2.1 around its axis n. At the same time the third motor L1 can rotate the guide rail P2.3 mounted on the first platform P2.1 around an axis n1, wherein the axis n and n1 are coaxial. Therefore the rotation of the first platform P2.1 around its axis n is possible as well as an additional rotation of the guide rail P2.3 around the axis n1. In this embodiment the second platform P2.4 is also equipped with a fourth motor L2 rotating and moving the second platform P2.4 along the guide rail P2.3 in direction G2. At the same time the second platform P2.4 is equipped with an intermediate platform L5, with which is connected a slidable platform L4 with mounted thereon first motor P2.6 with elements connected thereto. The intermediate platform L5 is also equipped with a fifth motor L3 executing reciprocating movement of the slidable platform L4 in direction G1. Equipping the tool arms assembly in subsequent motors L1, L2, L3 significantly enhances compensating movements of the surgical robot's arm by means of the tool arms. As a result, it is not necessary to use additional motors to drive the first tool arm P4. The support arm of the surgical robot controls the position of the first tool arm P4, whereas the motors L1, L2, L3 enable maintaining a stable point of the surgical instruments mounted to the arms P2a, P2b and maintaining its position while changing the position of the first tool arm P4.

## Claims

1. A surgical robot's tool arms assembly, comprising three movable tool arms (P4, P2a, P2b) mounted on a base (P1) provided for detachable fixing thereof on a support structure of a surgical robot, **characterised in that** the first tool arm (P4) is centrally mounted on the base (P1) by means of a rotatable joint (P4.3) and has a first guide rail (P4.2) with at least one arch-shaped guide groove (P4.2.1) and in said guide groove (P4.2.1) slides a second guide rail (P4.4) also having at least one arch-shaped guide groove (P4.4.1) in which slides a third guide rail (P4.5) arch-shaped in a side view, and said third guide rail (P4.5) is fixed to a base (P.4.8) for securing a surgical instrument, and at the same time the mutual position of all three guide rails (P4.2, P4.4, P4.5) is determined with a guiding and locking element (P4.6), preferably a screw mounted in the third guide rail (P4.5) and going through the guide grooves (P4.2.1, P4.4.1) and having a clamp (P4.7), whereas each of the two remaining tool arms (P2a, P2b) is situated on the opposite sides of the first tool arm (P4) and connected to the base (P1) with a first platform (P2.1) rotatably mounted on the base (P1) and each of the two tool arms (P2a, P2b) comprises a slidably and rotatably mounted to the first platform (P2.1) guide rail (P2.3) with a slidably and rotatably mounted thereon second platform (P2.4) locked on the guide rail (P2.3) with a locking element (P.5), preferably a screw, wherein on the second platform (P2.4) there is an immovably mounted first motor (P2.6.) and to a shaft thereof is mounted a first pivot bracket (P2.7) on a second end thereof there is an immovably mounted second motor (P2.8) and to the shaft of the second motor (P2.8) there is mounted a second pivot bracket (P2.9) provided for mounting thereon further surgical instrument.

2. The assembly according to claim 1, **characterised in that** the clamp (P4.7) of the screw (P4.6) locking the guide rails has eccentrically mounted therein roller elements (P4.7.1).

3. The assembly according to claim 1 or 2, **characterised in that** the second pivot bracket (P2.9) is connected with a linear slide mechanism (P2.10) and on said linear slide mechanism (P2.10) is mounted the surgical instrument (P3), wherein axes of rotation (o,p) of the first and the second motor (P2.4, P2.8) intersect at one point with an axis of rotation (r) of the surgical instrument (P3).

4. The assembly according to claim 1, **characterised in that** the fixing base (P4.8) has a form of a semi-open profile wherein the surgical instrument is slidably mounted.

5. The assembly according to one of claims 1-4, **characterised in that** the surgical instrument fixed to the fixing base (P4.8) of the first tool arm (P4) has a form of a video system body (P4.9) along which slides a tool slide (P4.11) with a camera (P5).

6. The assembly according to claim 1-5, **characterised in that** the first platform (P2.1) is equipped with a third motor (L1) enabling pivoting movement of the platform (P2.1) or the guide rail (P2.3) mounted thereon.

7. The assembly according to claims 1 - 6, **characterised in that** the second platform (P2.4) is equipped with a fourth motor (L2) enabling pivoting movement and movement of the platform along the guide rail (P2.3).

8. The assembly according to claims 1 - 7, **characterised in that** on the second platform (P2.4) there is mounted an intermediate platform (L5) with which a slidable platform (L4) is connected and on said slidable platform (L4) there is the immovably mounted first motor (P2.6).

9. The assembly according to claims 8, **characterised in that** the intermediate platform (L5) is equipped with a fifth motor (L3) enabling reciprocating movement of the slidable platform (L4).

## Patentansprüche

1. Anordnung von Werkzeugarmen eines chirurgischen Roboters mit drei beweglichen Werkzeugarmen (P4, P2a, P2b), eingebettet an einer Basis (P1), die zur lösbaren Befestigung an einer Trägerstruktur eines chirurgischen Roboters vorgesehen ist, **dadurch gekennzeichnet, dass** der erste Werkzeugarm (P4) zentral an der Basis (P1) mittels eines drehbaren Gelenks (P4.3) befestigt ist und eine erste Führung (P4.2) mit zumindest einer bogenförmigen Führungsrille (P4.2.1) aufweist, und in der Führungsrille (P4.2.1) eine zweite Führung (P4.4) mit zumindest einer bogenförmigen Führungsrille (P4.4.1) ausgeführt ist, in welcher eine dritte, in Seitenansicht einen gekrümmten Umriss aufweisende Führung (P4.5) gleitet und mit einer Basis (P4.8) für Befestigung eines chirurgischen Instruments verbunden ist, und gleichzeitig die gegenseitige Position aller drei Führungen (P4.2, P4.4, P4.5) mittels eines Führungs- und Verriegelungselements (P4.6) bestimmt wird, vorzugsweise einer in der dritten Führung (P4.5) montierten Schraube, die die Führungsrillen (P4.2.1, P4.4.1) von beiden verbleibenden Führungen durchquert und mit einem Druckelement (P4.7) ausgestattet ist, hingegen jeder der zwei verbleibenden Werkzeugarme (P2a, P2b) auf den entgegengesetzten Seiten des ersten Werkzeugarms (P4) angeordnet und mit der Basis (P1) durch eine erste mit der Basis (P1) drehbar befestigte Plattform (P2.1) verbunden ist, und jeder dieser beiden Werkzeugarme (P2a, P2b) eine verschiebbar und drehbar an der ersten Plattform (P2.1) gelagerte Führung (P2.3) mit einer daran verschiebbar und drehbar gelagerten zweiten Plattform (P2.4) aufweist, welche an der Führung (P2.3) mittels eines Verriegelungselements (P2.5), vorzugsweise einer Schraube, fixiert ist, wobei sich auf der zweiten Plattform (P2.4) ein unbeweglich gelagerter erster Motor (P2.6) befindet, an dessen Welle eine erste Drehhalterung (P2.7) befestigt ist, deren anderes Ende mit einem unbeweglich montierten zweiten Motor (P2.8) verbunden ist, und an die Welle des zweiten Motors (P2.8) eine zweite Drehhalterung (P2.9) für Montage eines chirurgischen Instruments befestigt ist.

2. Anordnung von Werkzeugarmen nach Anspruch 1, **dadurch gekennzeichnet, dass** das die Führungen verriegelnde Druckelement (P4.7) der Schraube (P4.6) mit exzentrisch gelagerten Wälzkörpem (P4.7.1) versehen ist.

3. Anordnung von Werkzeugarmen nach Anspruch 1 oder 2, **dadurch gekennzeichnet, dass** die zweite Drehhalterung (P2.9) mit einem linearen Schiebemechanismus (P2.10) verbunden ist, an dem ein chirurgisches Instrument (P3) befestigt ist, wobei sich Drehachsen (o, p) des ersten und des zweiten Motors (P2.4, P2.8) an einem Punkt mit einer Rotationsachse (r) des chirurgischen Instruments (P3) schneiden.

4. Anordnung von Werkzeugarmen nach Anspruch 1, **dadurch gekennzeichnet, dass** die Befestigungsbasis (P4.8) eine Form eines halboffenen Profils aufweist, in dem das chirurgische Instrument verschiebbar gelagert ist.

5. Anordnung von Werkzeugarmen nach einem der Ansprüche 1 -4, **dadurch gekennzeichnet, dass** das an die Befestigungsbasis (P4.8) des ersten Werkzeugarms (P4) befestigte chirurgische Instrument eine Form eines Videosystemkörpers (P4.9) aufweist, entlang dem ein Werkzeugschlitten (P4.11) mit einer Kamera (P5) gleitet.

6. Anordnung von Werkzeugarmen nach Anspruch 1 - 5, **dadurch gekennzeichnet, dass** die erste Plattform (P2.1) mit einem dritten Motor (L1) zum Drehen der Plattform (P2.1) beziehungsweise der daran befestigten Führung (P2.3) ausgestattet ist.

7. Anordnung von Werkzeugarmen nach Anspruch 1 - 6, **dadurch gekennzeichnet, dass** die zweite Plattform (P2.4) mit einem vierten Motor (L2) ausgestattet ist, der eine Drehbewegung und Bewegung der Plattform (P2.3) entlang der Führung ermöglicht.

8. Anordnung von Werkzeugarmen nach Anspruch 1 - 7, **dadurch gekennzeichnet, dass** auf der zweiten Plattform (P2.4) eine Zwischenplattform (L5) angebracht ist, mit der eine Schiebeplattform (L4) verbunden ist, an der der erste Motor (P2.6) unbeweglich befestigt ist.

9. Anordnung von Werkzeugarmen nach Anspruch 8, **dadurch gekennzeichnet, dass** die Zwischenplattform (L5) mit einem fünften Motor (L3) ausgestattet ist, der eine hin- und hergehende Bewegung der Schiebeplattform (L4) ausführt.

## Revendications

1. Groupe de bras porte-outils d'un robot chirurgical contenant trois bras porte-outils mobiles (P4, P2a, P2b) encastrés dans la base (P1) destinée à la fixation séparable de la construction de support d'un robot chirurgical **caractérisé en ce que** le premier bras porte-outils (P4) est encastré dans la base (P1) d'une manière centralisée à l'aide un joint tournant (P4.3) et qui contient la première glissière (P4.2), où se trouve au moins une rainure de guidage en forme d'arc (P4.2.1) où dans ladite rainure de guidage (P4.2.1) se déplace une deuxième glissière (P4.4) qui possède également au moins une rainure de guidage en forme d'arc (P4.1.1) dans laquelle se déplace une troisième glissière (P4.5) ayant le profil en forme d'arc de la vue de côté, et qui est liée à la base (P4.8) de fixation de l'appareil chirurgical et en même temps le positionnement réciproque de toutes les trois glissières (P4.2, P4.4, P4.5) est determiné par un élément de guidage et de blocage, profitablement à l'aide d'une vis placée sur la troisième glissière (P4.5), et qui passe par les rainures de guidage (P4.2.1, P4.4.1) des deux autres glissières et qui est équipée d'une bride (P4.7), cependant chacun des deux autres bras porte-outils (P2a, P2b) est situé sur les côtés opposés du premier bras porte-outils (P4) et est relié à la base (P1) par l'intermédiaire de la première plate-forme (P2.1) fixée d'une manière rotative sur la base (P1) et chacun des ces deux bras porte-outils (P2a, P2B) contient une glissière (P2.3) reliée d'une manière coulissante et rotative à la première plate-forme (P2.1), dans ladite glissière (P2.3) est encastré d'une manière coulissante et rotative une deuxième plate-forme (P2.4) qui est bloquée sur la glissière (P2.3) à l'aide de l'élément de blocage (P2.5), profitablement à l'aide d'une vis, cependant sur la deuxième plate-forme (P2.4) est fixé d'une manière immobile le premier moteur (P2.6) avec un arbre sur lequel est fixé le premier support rotatif (P2.7) et sur l'autre extrémité de l'arbre est fixé le deuxième moteur (P2.8) et à l'arbre du deuxième moteur (P2.8) est fixé un deuxième support rotatif (P2.9) qui sert à encastrer l'appareil chirurgical.

2. Groupe de bras selon la revendication 1 **caractérisé en ce que** la bride (P4.7) de la vis (P4.6) de blocage des glissières est équipée d'éléments roulants (P4.7.1) encastrés d'une manière excentrique.

3. Groupe de bras selon la revendication 1 ou 2 **caractérisé en ce que** le deuxième suport rotatif (P2.9) est relié au mécanisme de déplacement linéaire (P2.10), où est encastré un appareil chirurgical (P3) cependant les axes de rotation (o, p) du premier et du deuxième moteur (P2.4, P2.8) se coupent en un point avex l'axe de rotation (r) de l'appareil chirurgical (P3).

4. Groupe de bras selon la revendication 1, **caractérisé en ce que** la base de fixation (P4.8) a la forme d'un profilé semi-ouvert, où l'appareil chirurgical est fixé d'une manière coulissante.

5. Groupe de bras selon l'une des revendications 1 - 4, **caractérisé en ce que** l'appareil chirurgical fixé sur la base de fixation (P4.8) du premier bras porte - outils (P4) a la forme d'un corpus de la trajectoire de vision (P4.9) sur laquelle se déplace un chariot supérieur (P4.11) avec une camera (P5).

6. Groupe de bras selon les revendications 1 - 5, **caractérisé en ce que** la première plate-forme (P2.1) est équipée d'un troisième moteur (L1) qui réalise un mouvement rotatif de la plate-forme (P2.1) ou de la glissière fixée dessus. (P2.3).

7. Groupe de bras selon les revendications 1 - 6, **caractérisé en ce que** la deuxième plate-forme (P2.4) est équipée d'un quatrième moteur (L2) qui réalise un mouvement rotatif et un déplacement de la plate -forme le long de la glissière (P2.3).

8. Groupe de bras selon les revendications 1 - 7, **caractérisé en ce que** sur la deuxième plate-forme (P2.4) est encastré une plate-forme intermédiaire (L5) à qui est relié une plate-forme coulissante (L4) sur laquelle est fixé d'une manière immobile le premier moteur (P2.6).

9. Groupe de bras selon la revendication 8, **caractérisé en ce que** la plate-forme intermédiaire (L5) est équipée d'un cinquième moteur (L3) qui réalise un mouvement alternatif de la plate -forme coulissante (L4).
